# EUROPEAN PATENT APPLICATION

(11) **EP 4 752 146 A1**
(43) Date of publication of application: **03.06.2026**
(21) Application number: 24860504.0
(22) Date of filing: 02.09.2024
(51) Int. Cl.: C07D 491/22, A61K 47/68, A61P 35/00

(54) **NOVEL CAMPTOTHECIN DERIVATIVE AND CARRIER-DRUG CONJUGATE COMPRISING SAME**

(30) Priority: 31.08.2023 KR 20230115517
(71) Applicant: Pinotbio, Inc., Gyeonggi-do 16506 (KR)
(72) Inventor: JUNG, Doo Young, Suwon-si, Gyeonggi-do 16506 (KR); CHO, Hyun Yong, Suwon-si, Gyeonggi-do 16506 (KR); JUNG, Jin Kyo, Suwon-si, Gyeonggi-do 16506 (KR); MATHI, Gangadhar Rao, Suwon-si, Gyeonggi-do 16506 (KR)
(74) Representative: Prüfer & Partner mbB Patentanwälte · Rechtsanwälte
(86) International application number: PCT/KR2024/013194
(87) International publication number: WO 2025/048588

(57) **Abstract**

The present disclosure relates to a novel camptothecin derivative having a structure of Chemical Formula 1 and a carrier-drug conjugate comprising the same, wherein the novel camptothecin derivative has more desirable properties than conventional camptothecin derivatives in terms of efficacy, toxicity, selectivity, duration of action, administration, handling, stability, and/or manufacturability, and has the advantage of maximizing the efficacy of primary drug target modulation through (i) simultaneous inhibition of resistance proteins such as Survivin and other members of the Bcl family and/or (ii) inhibition of the activity of efflux pumps.

The novel camptothecin derivative according to the present disclosure and the carrier-drug conjugate comprising the same exhibit excellent anticancer efficacy and safety and may be used for the treatment of various cancers.

## Description

### FIELD

The present disclosure relates to a novel camptothecin derivative having excellent anticancer efficacy and safety, a carrier-drug conjugate comprising the same, and a pharmaceutical use thereof.

### BACKGROUND

Inhibitors of type I topoisomerase (Topoisomerase I), which is an isomerase involved in DNA replication, recombination, and the like, are drugs having an anticancer mechanism whose efficacy and safety have been validated, and have demonstrated excellent anticancer efficacy in various refractory solid tumors, such as colorectal cancer, lung cancer, breast cancer, and ovarian cancer, in clinical settings.

Representative type I topoisomerase inhibitors include camptothecin (CPT) and derivatives thereof. However, camptothecin has low aqueous solubility and exhibits high toxicity.

In order to address these problems, studies have been conducted on camptothecin derivatives having low toxicity and high aqueous solubility, and drugs such as irinotecan and topotecan have been approved and are currently marketed for the treatment of colorectal cancer, ovarian cancer, lung cancer, and the like.

To date, various camptothecin derivatives have been developed. Among them, SN-38 is a potent topoisomerase I inhibitor having IC₅₀ values in the nanomolar range in various cell lines, and is the active form of irinotecan, a prodrug used in the treatment of colorectal cancer, and also exhibits activity in lung cancer, breast cancer, and brain cancer. In the case of a Trop-2-SN-38 antibody-drug conjugate (ADC: Antibody-Drug Conjugate), efficacy has been demonstrated in multiple cancer types, including TNBC, bladder cancer, and gastric cancer; however, resistance to SN-38 still remains an issue.

In addition, Daiichi Sankyo employed DXd, a derivative of exatecan, which is a cytotoxic agent exhibiting approximately 10-fold higher activity in cancer cells than SN-38, in the development of Enhertu. DXd has good solubility, is relatively safe, and exhibits a high bystander killing effect, thereby providing advantages in the treatment of heterogeneous tumors; however, it has the disadvantage of a short half-life for reducing off-target effects.

When camptothecin-based drugs developed to date are used as payloads in antibody-drug conjugates (ADCs), they exhibit lower cytotoxicity compared to ADCs comprising conventional ultra-toxic payloads such as MMAE or MMAF, while still presenting safety issues. In addition, they suffer from reduced anticancer efficacy due to the development of resistance caused by overexpression of ABCG2 drug efflux pumps and the like.

Accordingly, there is an urgent need for camptothecin derivatives that exhibit higher cytotoxicity while maintaining excellent safety, and that significantly reduce resistance leading to decreased anticancer efficacy, particularly camptothecin derivatives suitable as payloads and carrier-drug conjugates comprising the same.

### SUMMARY OF THE DISCLOSURE

### OBJECTS OF THE DISCLOSURE

In view of the foregoing technical background, an object of the present disclosure is to provide a novel camptothecin derivative that exhibits a strong type I topoisomerase inhibitory effect, thereby demonstrating high cytotoxicity while maintaining excellent safety.

Another object of the present disclosure is to provide a carrier-drug conjugate comprising the camptothecin derivative according to the present disclosure, and a pharmaceutical composition comprising the camptothecin derivative according to the present disclosure or a carrier-drug conjugate comprising the same.

Still another object of the present disclosure is to provide a method for treating cancer using the camptothecin derivative according to the present disclosure or a carrier-drug conjugate comprising the same.

### TECHNICAL SOLUTION

In order to solve the above-described problems, a first aspect of the present disclosure provides a compound represented by Chemical Formula 1, an isomer thereof, a pharmaceutically acceptable salt thereof, a solvate thereof, or a prodrug thereof.

A second aspect of the present disclosure provides a pharmaceutical composition for preventing or treating cancer, comprising the compound represented by Chemical Formula 1, an isomer thereof, a pharmaceutically acceptable salt thereof, a solvate thereof, or a prodrug thereof.

Even when simple substituents such as C₁₋₃ alkyl, hydroxy, halogen, or amino groups are introduced into the compound of Chemical Formula 1 according to the present disclosure, or when existing hydroxy, ethyl, or oxo groups are substituted with other functional groups or are removed, such modifications are encompassed within the scope of equivalents of the present disclosure, provided that effects equivalent to those of the immunoconjugate of the present disclosure are achieved.

The present disclosure is described in further detail below.

The present disclosure provides a novel camptothecin derivative having the structure of Chemical Formula 1.

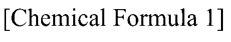

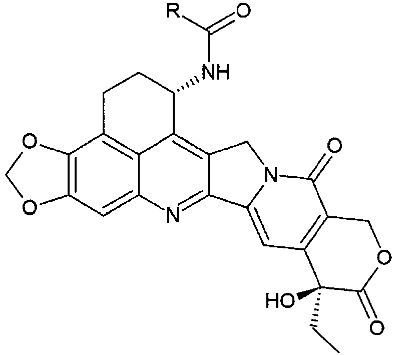

In Chemical Formula 1, R is selected from the group consisting of hydrogen; halogen; substituted or unsubstituted C1-C10 alkyl; substituted or unsubstituted C1-C10 alkoxy; substituted or unsubstituted C2-C10 alkenyl; C2-C10 alkynyl; substituted or unsubstituted C3-C10 cycloalkyl; substituted or unsubstituted C2-C10 heterocycloalkyl; substituted or unsubstituted C6-C10 aryl; substituted or unsubstituted C2-C10 heteroaryl; C1-C10 mono- or dialkylamino; nitro; and cyano.

In one embodiment, in Chemical Formula 1, R is selected from the group consisting of:

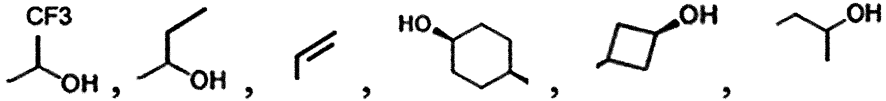

More specifically, the compound of Chemical Formula 1 may be a camptothecin derivative represented by Chemical Formulae 2 to 10 (referred to as Compounds 2 to 10, respectively), but is not limited thereto.

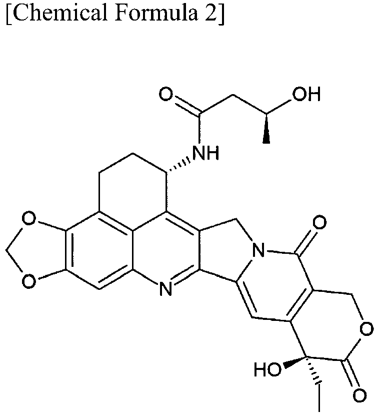

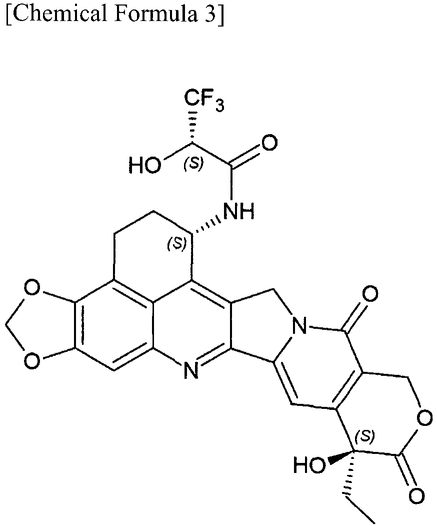

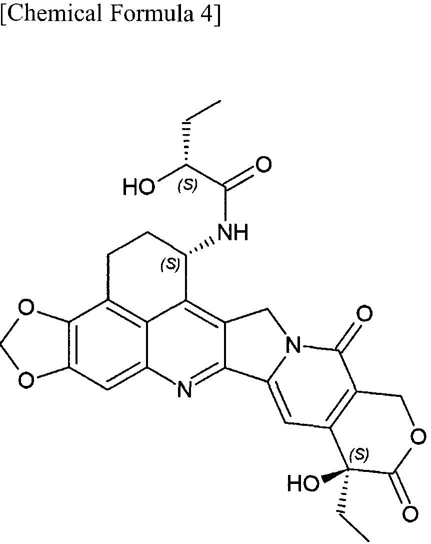

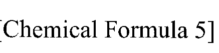

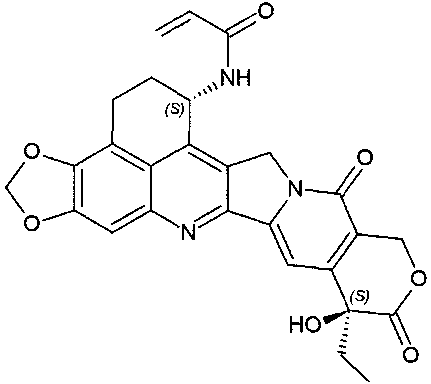

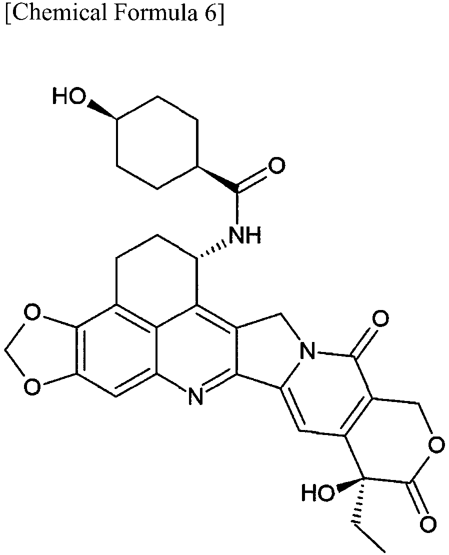

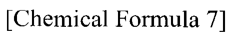

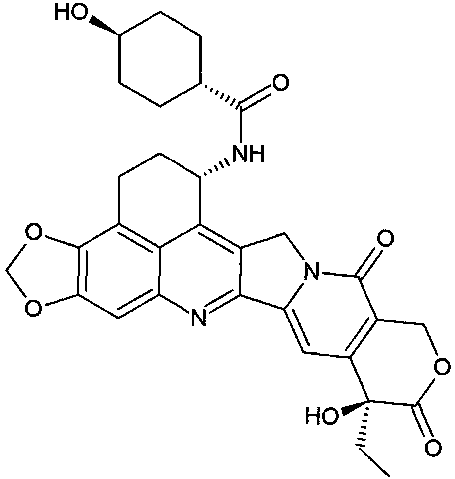

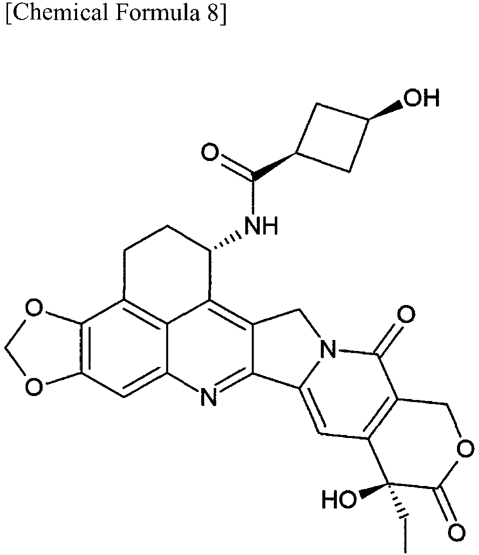

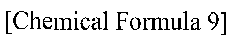

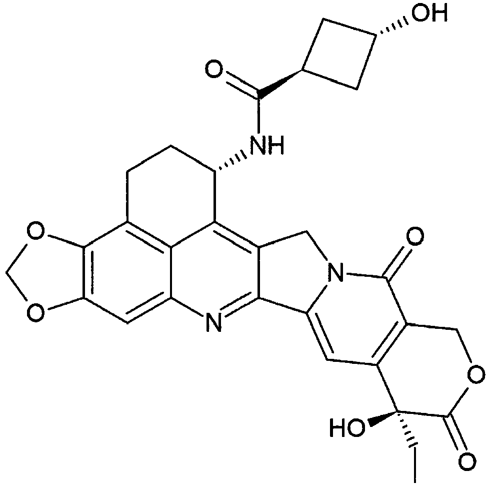

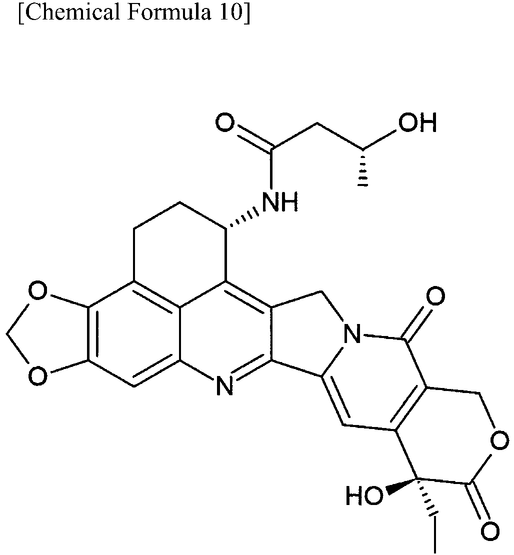

The camptothecin derivative represented by Chemical Formula 1 according to the present disclosure, or an isomer thereof, is a hydrophobic small molecule capable of permeating a cell membrane, and thus can be delivered to tumor tissue by the enhanced permeability and retention (EPR) effect, can penetrate deeply into tumor tissue and accumulate therein at a high concentration, can pass through the cell membrane to exert cytotoxicity within cells and induce cell death, and thereafter can be released and successively pass through the cell membrane of surrounding cells to move into the cells and exert its effect.

In the present specification, the camptothecin derivative represented by the compound of Chemical Formula 1 according to the present disclosure, or an isomer thereof, includes not only the compound of Chemical Formula 1 or an isomer thereof, but also a pharmaceutically acceptable salt thereof, a solvate thereof, and a prodrug thereof.

In the present specification, the pharmaceutically acceptable salt refers to salts commonly used in the pharmaceutical industry, and includes, for example, salts of inorganic ions such as sodium, potassium, calcium, magnesium, lithium, copper, manganese, zinc, and iron; salts of inorganic acids such as hydrochloric acid, phosphoric acid, and sulfuric acid; salts of organic acids such as ascorbic acid, citric acid, tartaric acid, lactic acid, maleic acid, malonic acid, fumaric acid, glycolic acid, succinic acid, propionic acid, acetic acid, orotic acid, and acetylsalicylic acid; and amino acid salts such as lysine, arginine, and guanidine. The pharmaceutically acceptable salt further includes salts of organic ions such as tetramethylammonium, tetraethylammonium, tetrapropylammonium, tetrabutylammonium, benzyltrimethylammonium, and benzethonium, which may be used in pharmaceutical reactions, purification, and separation processes, but is not limited thereto.

The term "prodrug" has the meaning commonly used in the relevant technical field. For example, a prodrug is an inactive compound that is converted into an active form in the body through drug metabolism. For example, it refers to a compound designed to chemically modify a physiologically active substance or a therapeutically active organic compound and to release or liberate a parent compound under enzymatic or other conditions in vivo. A prodrug is converted into the intended compound in the body after administration. Even when a compound is useful as a drug, if it has properties that are unsuitable in terms of side effects, stability, solubility, absorption, duration of action, or the like, chemical modification may be made thereto to enable clinical use.

The term "solvate" means a compound represented by Chemical Formula 1, or an isomer thereof, or a pharmaceutically acceptable salt thereof, further comprising a stoichiometric or non-stoichiometric amount of a solvent bound by non-covalent intermolecular forces. When the solvent is water, the solvate is a hydrate.

In one aspect, the present disclosure provides a carrier-drug conjugate comprising the compound represented by Chemical Formula 1 according to the present disclosure, an isomer thereof, a pharmaceutically acceptable salt thereof, or a solvate thereof.

In the present disclosure, the term "carrier" means a substance having the ability to selectively and specifically deliver the camptothecin derivative according to the present disclosure to a target site, for example, cancer cells, and may be an antibody, a peptide, a lipibody, and/or an aptamer, but is not limited thereto, and is preferably an antibody.

An aptamer-drug conjugate (ApDC) is obtained by introducing an aptamer in place of an antibody of an antibody-drug conjugate (ADC). An aptamer is a single-stranded nucleic acid having a three-dimensional structure. Aptamers are generally identified through a SELEX (Systematic Evolution of Ligands by Exponential Enrichment) process. SELEX is a technique for obtaining functional nucleic acids that bind to a target by introducing a protein molecule to be targeted into a compound library. Aptamers are capable of binding to a target very strongly and selectively and are therefore also referred to as chemical antibodies. Aptamers have a size of about 20 kDa and are known to have superior cell permeability and lower immunogenicity compared to antibodies.

Since aptamers can be chemically synthesized, precise design of the conjugation site and the number of conjugated drugs is possible in the preparation of an aptamer-drug conjugate. The production cost is lower than that of ADCs. Aptamers are generally composed of natural nucleic acids and are degraded by nucleases in vivo, resulting in low stability in the body. However, by taking advantage of the ease of chemical modification of aptamers, limitations in the stability of modified aptamers can be overcome.

A peptide-drug conjugate (PDC) is a form in which a peptide is introduced in place of an antibody in an antibody-drug conjugate (ADC). A peptide is composed of amino acids and has a size in the range of 500 to 5000 Da (Daltons). This is a very small size compared to an antibody having a size of 150 kDa (kilodaltons) or more. Accordingly, a peptide-based PDC has superior cell penetration ability compared to an ADC and has a very low possibility of inducing immunogenicity. In addition, peptides can be chemically synthesized. For this reason, a PDC not only has a very low production cost but also allows precise control of the conjugation site and ratio between the peptide and the drug. In general, peptides are easily degraded by proteolytic enzymes and thus have a short biological half-life. In order to overcome such limitations of peptide-based drug conjugates, strategies utilizing modified peptides, such as cyclic peptides and the introduction of non-natural amino acids, have been proposed.

A repebody is a type of artificial antibody that does not have an antibody scaffold but has a function of recognizing an antigen like an antibody. A repebody specific to a target protein can be identified through phage display technology.

Phage display is a technique for expressing a desired protein on the surface of a bacteriophage. A repebody has a size of about 30 kDa, which is approximately 20% of the size of an antibody. Accordingly, it is known to have relatively lower immunogenicity and improved cell permeability compared to an antibody. In addition, since the thermal and pH stability of a repebody can be adjusted, structural stability is expected to be improved. The production cost is also evaluated to be relatively lower than that of an antibody. Due to these advantages of repebodies, interest in the development of repebody-drug conjugates (Repebody-DC) as a strategy to replace antibodies with repebodies is increasing.

The target to which the carrier in the present disclosure binds, for example, an antigen, may include, without limitation, antigens selectively distributed on the surface of cancer cells, such as Her2, FolR, and PSMA, and cancer cell-overexpressed antigens that are present in small amounts in normal tissues, such as Trop2.

Exemplary cancer cell target antigens include 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOC1, AR, aromatase, ASPH, ATX, AX1, AXL, AZGP1 (zinc-a-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BIyS, BMP1, BMP2, BMP3B (GDFIO), BMP4, BMP6, BMP8, BMP10, BMPR1A, BMPR1B, BMPR2, BPAG1 (plectin), BRCA1, C19orf1O (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22(MDC/STC-I), CCL23 (MPIF-I), CCL24 (MPIF-2/eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNAl, CCNA2, CCNDl, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E-cadherin), CDH1O, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin-7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, Estrogen receptor, Earl, ESR2, F3 (TF), FADD, FAP, Farnesyltransferase, FasL, FASNf, FCER1A, FCER2, FCGR3A, FGF, FGF1 (aFGF), FGF10, FGF11, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (Fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, Histamine and Histamine receptor, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNBl, IFN gamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, IL1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (Glycoprotein 130), ILK, INHA, INHBA, INSL3, INSL4, IRAK1, IRAK2, ITGA1, ITGA2, ITGA3, ITGA6 (α6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (keratin 19), KRT2A, KRTHB6 (hair-specific type II keratin), L1CAM, LAG3, LAMA5, LAMP1, LEP (leptin), Lewis Y antigen ("LeY"), LILRB1, Lingo-p75, Lingo-Troy, LGALS3BP, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (metallothionein-UI), mTOR, MTSS1, MUC1 (mucin), MUC16, MYC, MYD88, NCK2, NCR3LG1, Neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P-cadherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, Phosphacan, PIAS2, PI3 kinase, PIK3CG, PLAU (uPA), PLG, PLXDCI, PKC, PKC-beta, PPBP (CXCL7), PPID, PR1, PRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (lipophilin B), SCGB2A1 (mammaglobin 2), SCGB2A2 (mammaglobin 1), SCYE1 (endothelial monocyte-activating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, Tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF1O (TRAIL), TNFRSF10A, TNFRSF10B, TNFRSF12A, TNFRSF17, TNFSF1 1 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll- like receptor, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, Versican, VHL C5, VLA-4, WT1, Wnt-1, XCL1 (lymphotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (dectin-2), CLEC5A (MDL-1, CLECSF5), CLEC1B (CLEC-2), CLEC9A (DNGR-1), CLEC7A (dectin-1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), or CH10 (Q64433), but is not limited thereto.

In addition, the target antigen may be an antigen that is distributed at least 10 times more abundantly in cancer cells than in normal cells.

In the present disclosure, non-limiting examples of the antibody include Urelumab, Utomilumab, Bebtelovimab, Aducanumab, Bapineuzumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab govitecan, Vobarilizumab, Cadonilimab, Vudalimab, Tebotelimab, Ivonescimab, Erfonrilimab, Ozoralizumab, Faricimab, Vanucizumab, or Navicixizumab, but are not limited thereto.

In the carrier-drug conjugate according to the present disclosure, it is preferable that the carrier and the drug are conjugated through a linker.

In the present disclosure, the linker should be stable in the bloodstream to prevent the drug from being separated from the carrier such as an antibody, thereby maintaining its structure until reaching a target such as an antigen so as to minimize damage to normal tissues. Ideally, the antibody-drug conjugate and the like should remain stable during systemic circulation, while being cleaved in target cells to appropriately release the cytotoxic drug, thereby safely delivering the drug to the target so that the antibody-drug conjugate and the like possess both efficacy and safety.

The carrier-drug conjugate according to the present disclosure may be characterized in that the carrier is conjugated, through a linker, to a drug, that is, a compound which is an active camptothecin derivative represented by Chemical Formula 1, preferably a compound of Chemical Formulae 2 to 10, but is not limited thereto.

In the carrier-drug conjugate according to the present disclosure, the compound which is an active camptothecin derivative represented by Chemical Formula 1, preferably a compound of Chemical Formulae 2 to 10, may be linked to the linker at an appropriate site so long as its properties such as anticancer activity are not changed. Accordingly, the present disclosure provides a drug-linker in which the compound which is an active camptothecin derivative represented by Chemical Formula 1, preferably a compound of Chemical Formulae 2 to 10, is linked to the linker.

Preferably, the drug-linker according to the present disclosure may have a structure of Chemical Formulae 2a to 10a, but is not limited thereto. In Chemical Formulae 2a to 10a, L means a linker.

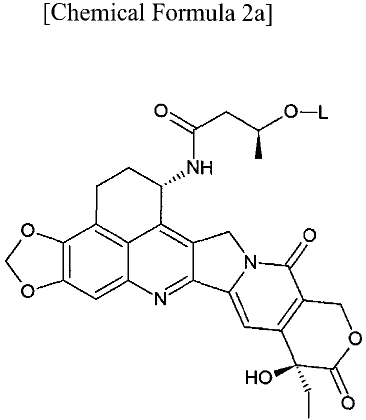

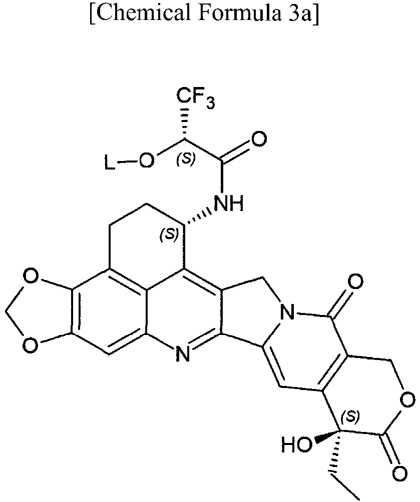

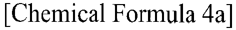

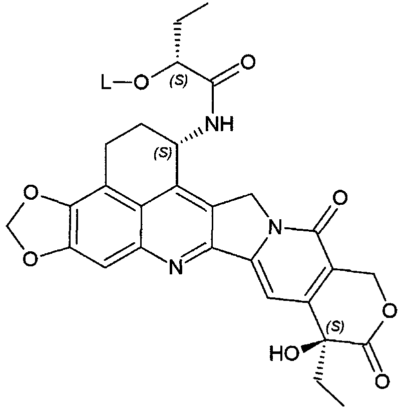

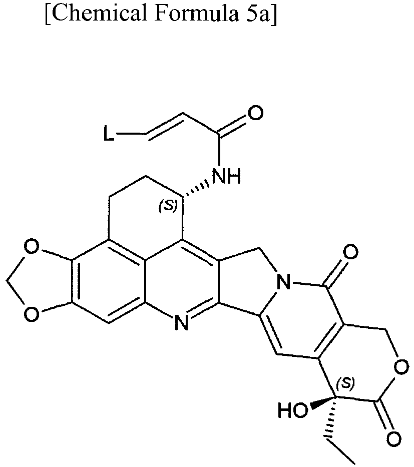

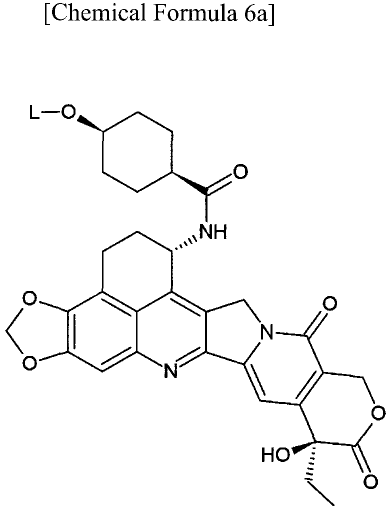

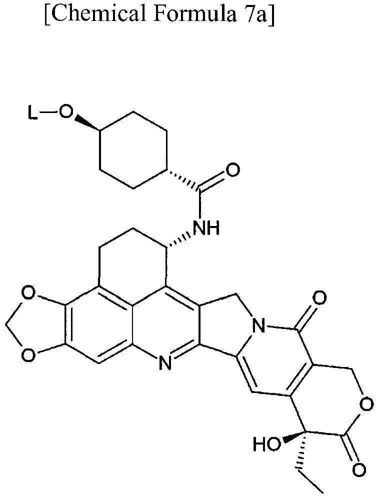

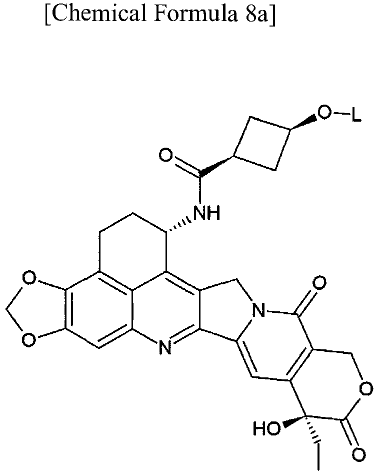

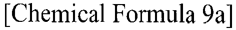

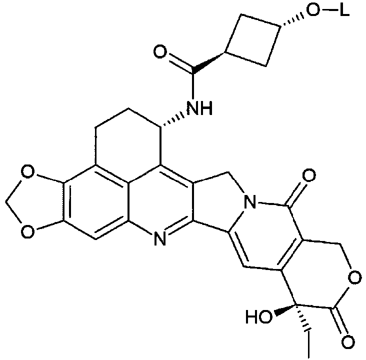

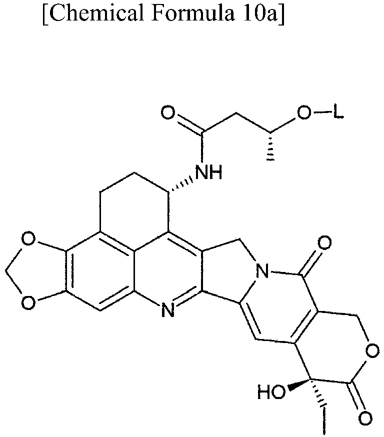

In the present disclosure, the linker may be in a form that is cleavable under a specific intracellular environment and/or condition, that is, in a form that allows the drug to be released from the antibody through cleavage of the linker in the intracellular environment.

For example, the linker can be cleaved by a cleaving agent present in an intracellular environment, for example, in a lysosome or an endosome, and may be a peptide linker that can be cleaved by an intracellular peptidase or protease enzyme, for example, a lysosomal or endosomal protease. In general, the peptide linker has a length of at least two or more amino acids. The cleaving agent may include cathepsin B, cathepsin D, and plasmin, and can hydrolyze the peptide to allow the drug to be released into a target cell. The peptide linker can be cleaved by the thiol-dependent protease cathepsin B, which is highly expressed in cancer tissues, and, for example, Gly-Gly-Phe-Gly (GGFG), Phe-Leu, or Gly-Phe-Leu-Gly linkers may be used, but are not limited thereto. In addition, the peptide linker may be one that can be cleaved by, for example, an intracellular protease, and may be a Val-Cit linker or a Phe-Lys linker.

In the present disclosure, the cleavable linker may be pH-sensitive and may be susceptible to hydrolysis at a specific pH value. In general, a pH-sensitive linker indicates that it can be hydrolyzed under acidic conditions. For example, the linker may be an acid-labile linker that can be hydrolyzed in a lysosome, such as hydrazone, semicarbazone, thiosemicarbazone, cis-aconitic amide, orthoester, acetal, or ketal.

In addition, in the present disclosure, the linker may also be cleaved under reducing conditions, and, for example, a disulfide linker may correspond thereto. Various disulfide bonds may be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alpha-methyl-alpha-(2-pyridyl-dithio)toluene).

The linker may include a beta-glucuronide linker that is recognized and hydrolyzed by beta-glucuronidase (β-glucuronidase), which is present in large amounts in lysosomes or overexpressed in certain tumor cells. Exemplarily, a beta-glucuronide linker disclosed in Korean Patent Laid-Open Publication No. 2015-0137015, for example, a beta-glucuronide linker comprising a self-immolative group, may be used.

In addition, the linker may be, for example, a non-cleavable linker, and the drug may be released through an antibody hydrolysis step to produce, for example, an amino acid-linker-drug complex. Such a type of linker may be a thioether group or a maleimidocaproyl group, and may maintain stability in blood.

Preferably, the linker according to the present disclosure may include GGFG. According to one embodiment of the present disclosure, a portion of at least one amino acid side chain constituting the linker may be substituted with a hydrophilic functional group that is cleavable under specific conditions. The hydrophilic functional group is preferably a monovalent hydrophilic functional group, and examples thereof may include beta-glucuronide or an ester or carbonate having a PEG (polyethylene glycol) group comprising 3 to 100 ethylene glycol repeating units, but are not limited thereto.

More preferably, the linker may have a structure of Chemical Formula 11 or Chemical Formula 12, but is not limited thereto.

| | |
|---|---|
| Chemical Formula 11 | 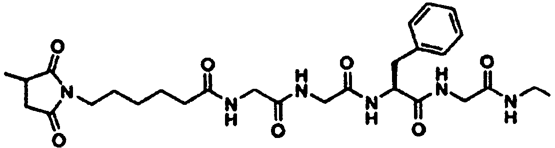 |
| Chemical Formula 12 | 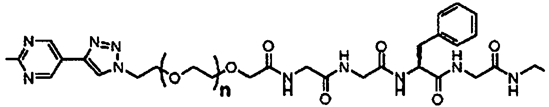 |

In Chemical Formula 12, n may be an integer of 3 to 10.

In the present disclosure, when the carrier in the carrier-drug conjugate is an antibody, the compound of Chemical Formula 2 to Chemical Formula 10, or the drug and/or drug-linker which is a compound of Chemical Formula 2a to Chemical Formula 10a, may be randomly conjugated through lysine residues in the antibody, or may be conjugated through cysteine residues exposed upon reduction of disulfide bond chains. In some cases, the linker-drug may be conjugated through lysine or cysteine present in a genetically engineered tag, for example, in a peptide or protein.

In addition, the present disclosure provides a pharmaceutical composition for preventing or treating cancer, comprising the compound represented by Chemical Formula 1 according to the present disclosure, or an isomer thereof, a pharmaceutically acceptable salt thereof, a solvate thereof, or a carrier-drug conjugate comprising the same.

Further, according to one specific embodiment of the present disclosure, there is provided a method for treating or preventing cancer, comprising administering to a subject in need thereof a therapeutically effective amount of the compound represented by Chemical Formula 1, or an isomer thereof, a pharmaceutically acceptable salt thereof, a solvate thereof, or a carrier-drug conjugate comprising the same. The subject may be a mammal, including a human.

In the present disclosure, the cancer includes any cancer that is treatable by inhibition of topoisomerase I and/or inhibition of one or more cancer-associated survival genes selected from the group consisting of survivin, Mcl-1, XIAP, and cIAP2, and may be a solid cancer or a hematologic cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, epithelial ovarian cancer, ovarian germ cell cancer, male breast cancer, brain cancer, pituitary adenoma, multiple myeloma, gallbladder cancer, bile duct cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary origin, gastric lymphoma, gastric cancer, gastric carcinoid tumor, gastrointestinal stromal tumor, Wilms tumor, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, acoustic neuroma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, hematologic cancer, and thymic cancer, but is not limited thereto. In addition, the cancer includes not only primary cancer but also metastatic cancer.

In the present specification, the terms "patient," "subject," and "individual" refer to an animal such as a mammal. In certain embodiments, the patient is a human. In other embodiments, the patient is a non-human animal such as a dog, a cat, livestock (for example, a horse, a pig, or a donkey), a chimpanzee, or a monkey.

As used herein, the term "therapeutically effective amount" refers to an amount of the compound represented by Chemical Formula 1, or an isomer thereof, a pharmaceutically acceptable salt thereof, a solvate thereof, or a carrier-drug conjugate comprising the same, that is effective for treating or preventing cancer. Specifically, the term "therapeutically effective amount" means an amount sufficient to treat a disease at a reasonable benefit/risk ratio applicable to medical treatment, and the effective dosage level may be determined depending on factors including the type and severity of the subject, age, sex, type of disease, activity of the drug, sensitivity to the drug, time of administration, route of administration, rate of excretion, duration of treatment, concomitantly administered drugs, and other factors well known in the medical field. The pharmaceutical composition of the present disclosure may be administered as a single therapeutic agent or in combination with other therapeutic agents, and may be administered sequentially or simultaneously with commercially available therapeutic agents. The pharmaceutical composition of the present disclosure may be administered as a single dose or in multiple doses. It is important to administer an amount that achieves the maximum effect with the minimum amount without side effects in consideration of all of the above factors. Since the compound represented by Chemical Formula 1, or an isomer thereof or a pharmaceutically acceptable salt thereof, or a carrier-drug conjugate comprising the same exhibits a dose-dependent effect, the dosage may be readily determined by those skilled in the art according to various factors such as the patient's condition, age, sex, and complications. Since the active ingredient of the pharmaceutical composition of the present disclosure has excellent safety, it may be used at a dose exceeding the determined dosage.

Further, according to one specific embodiment of the present disclosure, there is provided a use of the compound represented by any one of Chemical Formulae 1 to 10, or an isomer thereof, a pharmaceutically acceptable salt thereof, a solvate thereof, or a carrier-drug conjugate comprising the same, for the manufacture of a medicament for use in the treatment or prevention of cancer.

The compound represented by any one of Chemical Formulae 1 to 10, or an isomer thereof, a pharmaceutically acceptable salt thereof, a solvate thereof, or a carrier-drug conjugate comprising the same, for the manufacture of a medicament may be mixed with pharmaceutically acceptable adjuvants, diluents, carriers, and the like, and may be formulated as a combined preparation together with other active agents to provide synergistic effects of the active ingredients.

The matters described with respect to the use, composition, and method of treatment of the present disclosure are equally applicable to one another unless inconsistent therewith.

In the present specification, the anticancer effect or therapeutic effect of an anticancer agent may refer to an action that reduces the severity of cancer, reduces tumor size, or delays or slows the progression of cancer occurring while a patient is suffering from a particular cancer.

For example, the anticancer effect of an anticancer agent may be cell viability (the degree of cytotoxicity or a change in cell number) of cancer cells after treatment of the cancer cells with the anticancer agent in vitro and/or in vivo. For example, it may be indirectly confirmed through a drug response test using a cell line or a non-clinical animal model (xenograft). In addition, in cancer patients, the anticancer effect of an anticancer agent may be directly confirmed, and data related thereto may be derived and used as a database. Further, in designing a dosing guideline for an anticancer agent, animal model PK parameters and/or toxicity profiles may be considered in parallel.

The anticancer effect of an anticancer agent may also be inferred from in vitro data, such as the % maximum effect of the anticancer agent, for example IC₅₀, IC₆₀, IC₇₀, IC₈₀, and IC₉₀, and may be confirmed in non-clinical animal models and clinical cancer patients through in vivo data such as the maximum blood concentration (Cmax) and/or the area under the blood drug concentration-time curve (AUC) of the drug.

The responsiveness of an anticancer agent means clinical sensitivity in terms of anticancer effect.

In the context of treatment using an anticancer agent, the terms "sensitivity" and "sensitive" are relative terms referring to the degree of the effect of a compound in alleviating or reducing the progression of a treated tumor or disease.

An "effective anticancer effect/response in a patient" may be, for example, inhibition of 5%, 10%, 15%, 20%, 25%, 30%, 40%, 50%, 60%, 70%, 80%, 90%, 100%, 200%, or more in a patient response, as measured by any suitable means, for example gene expression, cell counting, or assay results.

In the present specification, the administration dose is a dose at which a pharmacological effect is expected. In the present disclosure, the pharmacological effect may be an anticancer effect. The responsiveness (anticancer effect) of an anticancer agent may be expressed as the degree of response, and may be the % maximum effect of the anticancer agent, for example IC₅₀, IC₆₀, IC₇₀, IC₈₀, and IC₉₀, or a value (LC₅₀) at which toxicity to normal cells is exhibited.

For example, an oral formulation may be formulated using various formulation techniques known in the art. For example, it may include a biodegradable (hydrolyzable) polymeric carrier used for adhesion to the oral mucosa. It may be designed to be gradually eroded over a predetermined period, wherein drug delivery is essentially provided overall.

In an oral formulation, drug delivery avoids drawbacks encountered in oral drug administration, for example, slow absorption, degradation of the active agent by fluids present in the gastrointestinal tract, and/or first-pass inactivation in the liver. With respect to a biodegradable (hydrolyzable) polymeric carrier, substantially any such carrier may be used as long as the desired drug release profile is not impaired, and the carrier is compatible with any other component present in the oral dosage unit. In general, the polymeric carrier includes a hydrophilic (water-soluble and water-swellable) polymer that adheres to the moist surface of the oral mucosa. An example of a polymeric carrier useful in the present specification is an acrylic acid polymer (e.g., carbomer). In some embodiments, non-limiting examples of other components that may be incorporated into the oral formulation include disintegrants, diluents, binders, lubricants, flavoring agents, coloring agents, and preservatives. In some embodiments, for oral or sublingual administration, it may be in the form of a tablet, lozenge, or gel formulated in a conventional manner.

In some embodiments, when the condition of the patient is improved, administration of the compound is continued at the discretion of a physician; alternatively, the dose of the drug to be administered may be temporarily reduced or temporarily discontinued for a certain period of time (i.e., a "drug holiday"). The length of the drug holiday may vary between 2 days and 1 year, and may include, by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. In some embodiments, the dose reduction during the drug holiday is 10%-100%, and includes, by way of example only, 10%, 15%, 20%, 25%, 30%, 35%, 40%, 45%, 50%, 55%, 60%, 65%, 70%, 75%, 80%, 85%, 90%, 95%, or 100%.

When improvement of the patient's condition occurs, a maintenance dose is administered, if necessary. Thereafter, the dosage or frequency of administration, or both, may be reduced as a function of symptoms to a level at which the improved disease, disorder, or condition is maintained. However, the patient may require intermittent treatment over an extended period of time upon any recurrence of symptoms.

The amount of a given formulation corresponding to such an amount will vary depending on factors of the subject in need of treatment, such as the particular compound and the severity of the disease and the identity (for example, body weight), but may nevertheless be routinely determined in a manner known in the art according to, for example, the formulation to be administered, the route of administration, and the specific circumstances surrounding the subject to be treated. In general, however, the dosage used for treatment of an adult human will typically range from 0.02-5000 mg/day, or about 1-1500 mg/day.

In the present specification, a single administration dose may be provided as a single dose or as divided doses administered simultaneously, for example, as 2, 3, 4, or more sub-doses.

In some embodiments, an oral formulation is a unit dosage form suitable for single administration of an exact dose. In the unit dosage form, the formulation is divided into unit doses containing an appropriate amount of one or more compounds. In some embodiments, the unit dosage is in the form of a package containing discrete amounts of the formulation. Non-limiting examples include packaged tablets or capsules, and powder vials or ampoules. An aqueous suspension composition may be packaged in a single-dose non-reclosable container. Alternatively, a multi-dose reclosable container may be used, in which case it is typical to include a preservative in the composition.

In some embodiments, a parenteral injectable formulation is provided in a unit dosage form, including, without limitation, ampoules, together with added preservatives, or in a multi-dose container.

Typically, it is prepared for parenteral administration, that is, bolus, intravenous, and intratumoral injection, in a unit-dose injectable form together with a pharmaceutically acceptable parenteral vehicle. It is optionally mixed in the form of a lyophilized preparation or an aqueous solution together with pharmaceutically acceptable diluents, carriers, excipients, or stabilizers (Remington's Pharmaceutical Sciences (1980) 16th edition, Osol, A. Ed.).

### ADVANTAGEOUS EFFECTS

The camptothecin derivative represented by Chemical Formula 1 according to the present disclosure has more desirable properties than existing camptothecin derivatives in terms of efficacy, toxicity, selectivity, duration of action, administration, handling, stability, and/or manufacturability, and has an advantage in that it can maximize the efficacy of modulation of the main drug target through (i) simultaneous inhibition of Bcl family such as the resistance protein Survivin and/or (ii) an additional mechanism of action of inhibiting the activity of an efflux pump.

Furthermore, a carrier-drug conjugate comprising the camptothecin derivative represented by Chemical Formula 1 according to the present disclosure has an advantage in that it provides high safety by minimizing toxicity while simultaneously exhibiting high therapeutic efficacy.

The present disclosure provides compounds of Chemical Formulae 2 to 10 as examples of compounds corresponding to Chemical Formula 1, and among these, in the case of the compound of Chemical Formula 2, the compound alone or when prepared in the form of an ADC has high toxicity and selectivity toward cancer cells.

### BRIEF DESCRIPTION OF THE DRAWINGS

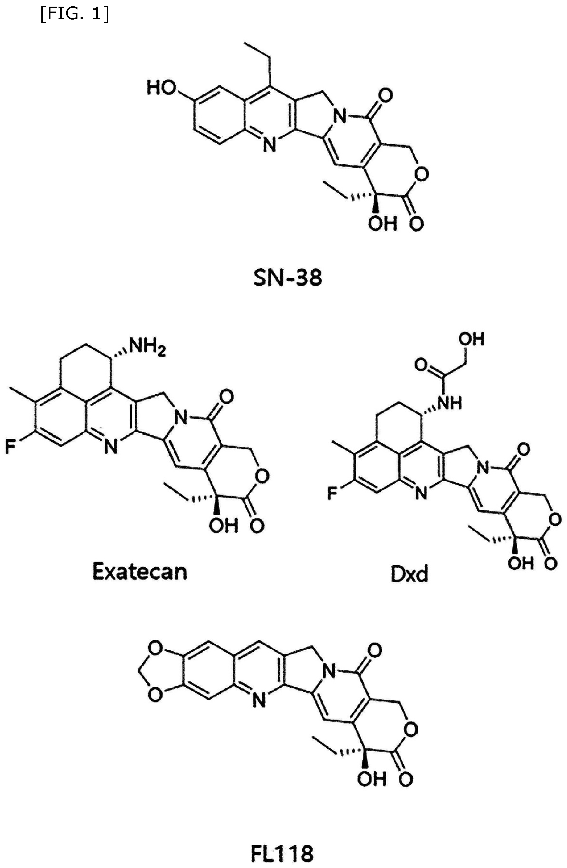

FIG. 1 is a drawing showing the structural formulas of various camptothecin-based anticancer agents (SN-38, Exatecan, Dxd, and FL118).
FIG. 2 shows the results of an in vitro cell viability assay of Compound 2 in the FaDu cell line and the NHEK cell line.
FIG. 3 shows the results of an in vitro cell viability assay of Compound 2 in the KPL-4 cell line.
FIG. 4 shows the results of an in vitro cell viability assay of Compound 2 in the MDA-MB-453 cell line.
FIG. 5 shows the results of an in vitro cell viability assay of Compound 2 in the MDA-MB-468 cell line.
FIG. 6 shows the results of a bystander killing effect analysis of a Compound 2-based ADC in the MDA-MB-468 and KPL-4 cell lines.
FIG. 7 shows the results of an in vitro cell viability assay of a Compound 2-based ADC in the MDA-MB-453 cell line.
FIG. 8 shows the results of an in vitro cell viability assay of a Compound 2-based ADC in the MDA-MB-468 cell line.

### BEST MODE FOR CARRYING OUT THE INVENTION

Hereinafter, the present disclosure will be described in more detail through Examples. However, the following Examples are provided only to clearly illustrate the technical features of the present disclosure and are not intended to limit the scope of protection of the present disclosure.

### [Example 1]

### Preparation of Materials

In the present disclosure, FaDu, A549, NHEK KPL-4, MDA-MB-453, and MDA-MB-468 used herein were purchased from ATCC; Trastuzumab was purchased from Wuxi Biologics; Enhertu was purchased from Daiichi-Sankyo; T-DM1 was purchased from Genentech; and Dxd was purchased from Symeres.

### Analysis Conditions

Analysis condition: U_AN_ACID, Agilent Infinity II; Bin. Pump: G7120A, Multisampler, VTC, DAD: Agilent G7117B, 220-320nm, PDA: 210-320nm, MSD: Agilent G6135B ESI, pos/neg 100-1000, ELSD G7102A: Evap 40°C, Neb 40°C, gas flow 1.6 mL/min; Column: Waters XSelect CSH C18, 50x2.1 mm, 2.5 µm, Temp: 40°C, Flow: 0.6 mL/min, Gradient: t0 = 5% B, t2min = 98% B, t2.7min = 98% B, Post time: 0.3 min, Eluent A: 0.1% formic acid in water; Eluent B: 0.1% formic acid in acetonitrile.

### Acidic Preparative MPLC (Luna)

Instrument type: Reveleris^{™} prep MPLC; Column: Phenomenex LUNA C18(3) (150x25mm, 10µ); Flow: 40mL/min; Column temp: room temperature; Eluent A: 0.1% (v/v) formic acid in water; Eluent B: 0.1% (v/v) formic acid in acetonitrile; Gradient; Detection UV: 220, 254, 340 nm, ELSD.

### Acidic Preparative MPLC (Reprosil)

Instrument type: Reveleris^{™} prep MPLC; Column: Dr. Maisch Reprosil C18 150x25 mm, 10µ); Flow: 40 mL/min; Column temp: room temperature; Eluent A: 0.1% (v/v) formic acid in water; Eluent B: 0.1% (v/v) formic acid in acetonitrile; Gradient; Detection UV : 220, 254, 340 nm, ELSD.

### Synthesis of Camptothecin Derivative

### Example 1-1. Synthesis of the Camptothecin Derivative of Chemical Formula 2 (Compound 2)

(S)-3-hydroxybutyric acid (19 mg, 0.18 mmol) was dissolved in 1 mL of N,N-dimethylformamide, and HATU (69 mg, 0.18 mmol) was added thereto, and the reaction mixture was stirred at room temperature for 20 minutes. 0.55 mL of this solution was added to a suspension prepared by dissolving (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione methanesulfonate (49 mg, 90 µmol) and DIPEA (39 µL, 0.23 mmol) in N,N-dimethylformamide (2.5 mL). The mixture was stirred at room temperature for 30 minutes and then stored at -20°C overnight. The reaction mixture was purified by acidic preparative MPLC (Luna10-50), and the product fractions were lyophilized to obtain a pale yellow solid. (Yield: 35 mg, 73%)

U_AN_ACID: m/z 534.2 [M+H]⁺

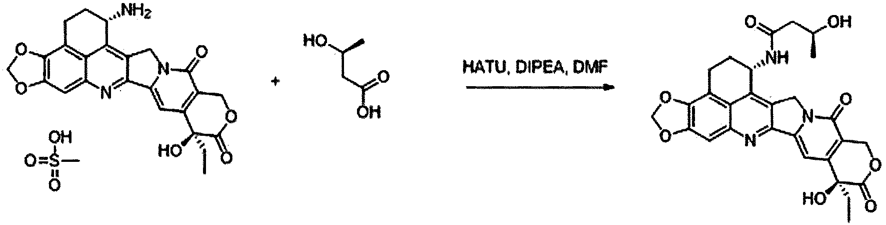

¹H NMR (400 MHz, DMSO-d6) δ 8.40 (d, J = 8.7 Hz, 1H), 7.42 (s, 1H), 7.24 (s, 1H), 6.48 (s, 1H), 6.29 (d, J = 8.7 Hz, 2H), 5.58 - 5.50 (m, 1H), 5.46 - 5.35 (m, 2H), 5.25 - 5.11 (m, 2H), 4.62 (d, J = 4.4 Hz, 1H), 4.10 - 3.98 (m, 1H), 3.08 - 2.98 (m, 2H), 2.28 (dd, J = 13.6, 7.4 Hz, 1H), 2.18 (dd, J = 13.6, 5.9 Hz, 1H), 2.14 - 1.99 (m, 2H), 1.92 - 1.77 (m, J = 7.1 Hz, 2H), 1.08 (d, J = 6.2 Hz, 3H), 0.87 (t, J = 7.3 Hz, 3H).

### Example 1-2. Synthesis of the Camptothecin Derivative of Chemical Formula 3 (Compound 3)

(S)-3,3,3-trifluoro-2-hydroxypropanoic acid (11.59 mg, 0.080 mmol) was dissolved in N,N-dimethylformamide (2 mL), and HATU (30.6 mg, 0.080 mmol) was added thereto.

After stirring the mixture for 3 minutes, (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (30 mg, 0.067 mmol) and DIPEA (0.047 mL, 0.268 mmol) were added, and the reaction mixture was stirred at room temperature for 1.5 hours. The reaction mixture was directly purified by acidic preparative MPLC (Luna10-50), and the product fractions were lyophilized to obtain an off-white solid. (Yield: 19 mg, 49%)

The compound (1S,10S)-1-amino-10-ethyl-10-hydroxy-1,2,3,10,13,16- 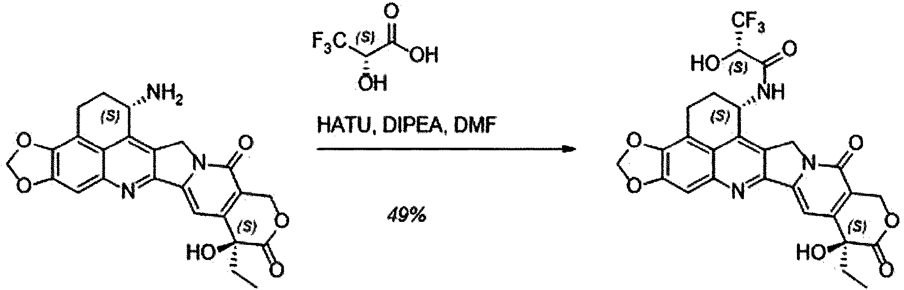 hexahydro-11H,14H-benzo[de][1,3]dioxolo[4,5-g]pyrano[3',4':6,7]indolizino[1,2-b]quinoline-11,14-dione may be obtained by a method described in PCT/KR2023/009854, but is not limited thereto.
U_AN_ACID: m/z 574.2 [M+H]⁺
¹H NMR (400 MHz, DMSO-d6) δ 8.95 (d, J = 8.6 Hz, 1H), 7.42 (s, 1H), 7.24 (s, 1H), 7.18 (d, J = 6.2 Hz, 1H), 6.48 (s, 1H), 6.29 (d, J = 7.1 Hz, 2H), 5.61 - 5.52 (m, 1H), 5.46 - 5.35 (m, 2H), 5.21 (d, J = 19.2 Hz, 1H), 5.06 (d, J = 19.2 Hz, 1H), 4.66 - 4.55 (m, 1H), 3.08 - 2.97 (m, 2H), 2.18 - 2.03 (m, 2H), 1.93 - 1.77 (m, 2H), 0.87 (t, J = 7.3 Hz, 3H).

### Example 1-3. Synthesis of the Camptothecin Derivative of Chemical Formula 4 (Compound 4)

(R)-2-hydroxybutyric acid (20.9 mg, 0.201 mmol) was dissolved in 1 mL of DMF. HOSu (23.1 mg, 0.201 mmol) and EDC (38.6 mg, 0.201 mmol) were added, and the reaction mixture was stirred at room temperature for 2 hours.

Then, 0.3 mL of the activated acid solution was added to a suspension prepared by dissolving (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (30 mg, 0.067 mmol) and triethylamine (0.019 mL, 0.134 mmol) in N,N-dimethylformamide (2 mL). The mixture was stirred at room temperature for 23 hours. The reaction mixture was directly purified by acidic preparative MPLC (Luna10-50), and the product fractions were lyophilized to provide an off-white solid. (Yield: 25 mg, 69%) 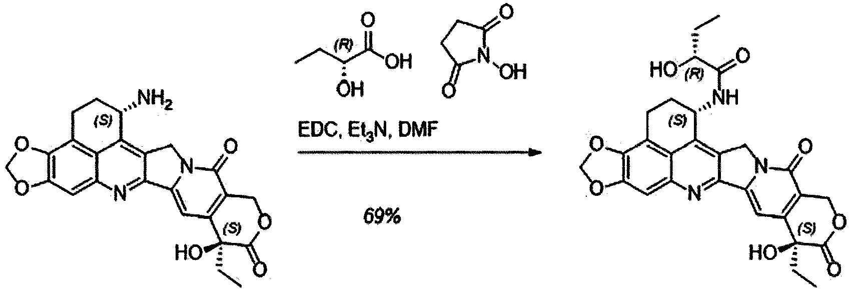
U_AN_ACID: m/z 534.2 [M+H]⁺
¹H NMR (400 MHz, DMSO) δ 8.39 (d, J = 8.9 Hz, 1H), 7.41 (s, 1H), 7.23 (s, 1H), 6.48 (s, 1H), 6.29 (d, J = 3.6 Hz, 2H), 5.57 - 5.50 (m, 1H), 5.48 - 5.44 (m, 1H), 5.41 (s, 2H), 5.13 (s, 2H), 3.92 - 3.86 (m, 1H), 3.06 - 3.00 (m, 2H), 2.15 - 2.05 (m, 2H), 1.92 - 1.74 (m, 3H), 1.71 - 1.62 (m, 1H), 0.93 (t, J = 7.4 Hz, 3H), 0.87 (t, J = 7.3 Hz, 3H).

### Example 1-4. Synthesis of the Camptothecin Derivative of Chemical Formula 5 (Compound 5)

A suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (30 mg, 0.067 mmol) and DIPEA (0.047 mL, 0.268 mmol) in dichloromethane (dry)(1 mL) was cooled to 0°C. A solution of acryloyl chloride (5.44 µL, 0.067 mmol) in DCM (0.1 mL) was added dropwise thereto. The reaction mixture was stirred at 0°C, and after 30 minutes, 0.15 equivalents of acryloyl chloride was added as a stock solution in DCM. After stirring for a total of 2 hours, the reaction mixture was concentrated under reduced pressure. The residue was dissolved in DMSO and purified by acidic preparative MPLC (Reprosil10-50 method). The product fractions were collected and lyophilized to obtain a pale yellow fluffy solid. (Yield: 22 mg, 65%)

U_AN_ACID: m/z 502.2 [M+H]⁺

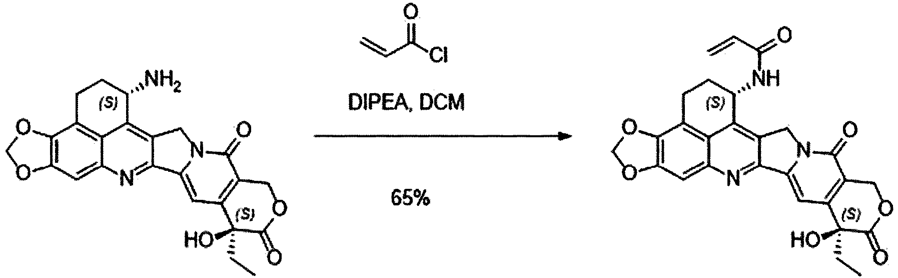

¹H NMR (400 MHz, DMSO-d6) δ 8.71 (d, J = 8.8 Hz, 1H), 7.43 (s, 1H), 7.23 (s, 1H), 6.49 (s, 1H), 6.30 (d, J = 4.2 Hz, 2H), 6.28 - 6.21 (m, 2H), 5.69 (dd, J = 8.5, 3.7 Hz, 1H), 5.65 - 5.58 (m, 1H), 5.40 (s, 2H), 5.25 - 5.12 (m, 1H), 5.12 - 4.99 (m, 1H), 3.10 - 3.02 (m, 2H), 2.16 - 2.06 (m, 2H), 1.92 - 1.78 (m, 2H), 0.86 (t, J = 7.3 Hz, 3H).

### Example 1-5. Synthesis of the Camptothecin Derivative of Chemical Formula 6 (Compound 6)

(1S,4S)-4-hydroxycyclohexane-1-carboxylic acid (11 mg, 1 Eq, 78 µmol) was dissolved in N,N-dimethylformamide (1 mL). (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (35 mg, 8 µmol), DIPEA (41 µL, 0.23 mmol), and HATU (30 mg, 1 Eq, 78 µmol) were added thereto. The reaction mixture was stirred at room temperature for 1 hour. The mixture was purified by acidic preparative MPLC (Luna5-40), and the product fractions were lyophilized to obtain a bright yellow solid. (Yield: 28 mg, 62%)

U_AN_ACID: m/z 574.2 [M+H]⁺

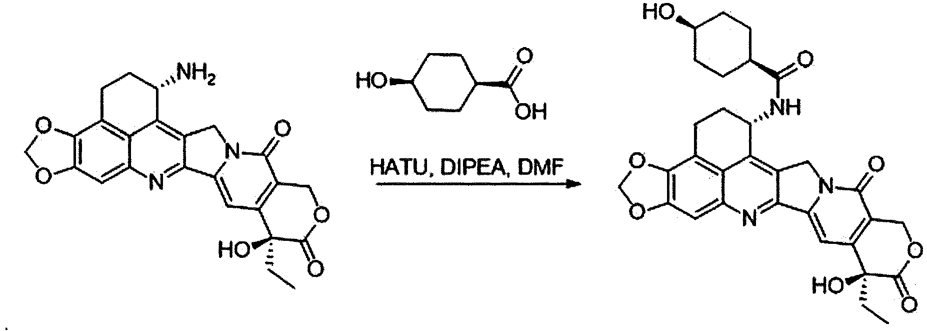

¹H NMR (400 MHz, DMSO-d6) δ 8.34 (d, J = 8.8 Hz, 1H), 7.41 (s, 1H), 7.23 (s, 1H), 6.50 (s, 1H), 6.29 (d, J = 6.4 Hz, 2H), 5.57 - 5.48 (m, 1H), 5.46 - 5.35 (m, 2H), 5.19 - 5.10 (m, 1H), 5.09 - 4.99 (m, 1H), 4.34 (d, J = 2.9 Hz, 1H), 3.77 (s, 1H), 3.10 - 2.97 (m, 2H), 2.24 - 2.13 (m, 1H), 2.10 - 1.99 (m, 2H), 1.93 - 1.78 (m, 4H), 1.72 - 1.60 (m, 2H), 1.55 - 1.34 (m, 4H), 0.87 (t, J = 7.3 Hz, 3H).

### Example 1-6. Synthesis of the Camptothecin Derivative of Chemical Formula 7 (Compound 7)

(1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (50 mg, 0.11 mmol) was suspended in N,N-dimethylformamide (2.5 mL), and DIPEA (78 µL, 0.45 mmol), (1R,4R)-4-hydroxycyclohexane-1-carboxylic acid (16 mg, 0.11 mmol), and HATU (42 mg, 1 Eq, 0.11 mmol) were added thereto. The reaction mixture was stirred at room temperature for 30 minutes. The reaction mixture was purified by acidic preparative MPLC (Luna5-40), and the product fractions were lyophilized. (Yield: 32 mg, 48%) 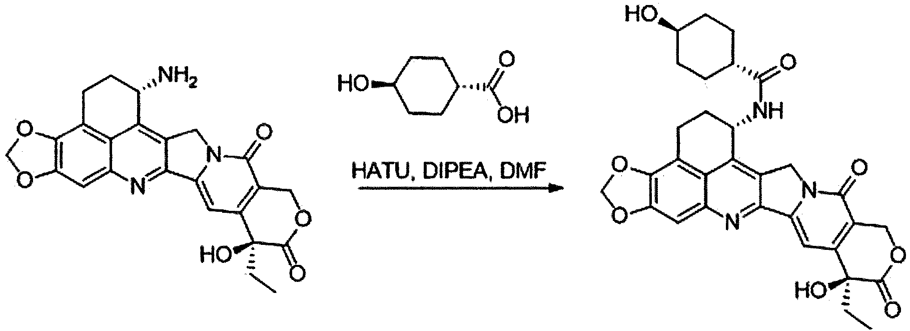
U_AN_ACID: m/z 574.2 [M+H]⁺
¹H NMR (400 MHz, DMSO-d6) δ 8.39 (d, J = 8.8 Hz, 1H), 7.41 (s, 1H), 7.22 (s, 1H), 6.50 (s, 1H), 6.29 (d, J = 6.6 Hz, 2H), 5.55 - 5.35 (m, 3H), 5.18 - 4.95 (m, 2H), 4.56 (d, J = 4.4 Hz, 1H), 3.11 - 2.97 (m, 2H), 2.15 - 1.98 (m, 3H), 1.94 - 1.71 (m, 6H), 1.54 - 1.39 (m, 2H), 1.16 - 1.00 (m, 2H), 0.87 (t, J = 7.3 Hz, 3H).

### Example 1-7. Synthesis of the Camptothecin Derivative of Chemical Formula 8 (Compound 8)

(1S,3S)-3-hydroxycyclobutane-1-carboxylic acid (21 mg, 0.18 mmol) was dissolved in N,N-dimethylformamide (2 mL), and HATU (70 mg, 0.18 mmol) was added. The reaction mixture was stirred for 1 hour. 1 mL of this solution was added to a suspension prepared by dissolving (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione methanesulfonate (50 mg, 92 µmol) and DIPEA (32 µL, 0.18 mmol) in N,N-dimethylformamide (2.5 mL). The reaction mixture was stirred at room temperature for 2 hours. An additional 0.15 mL of the activated acid solution was added, and after 20 minutes, DIPEA (16 µL, 92 µmol) was added. After a further 20 minutes, upon reaching complete conversion, the reaction mixture was loaded into a syringe and filtered, and then purified by acidic preparative MPLC (Luna5-40). The product fractions were lyophilized to obtain a pale yellow fluffy solid. (Yield: 30 mg, 60%) 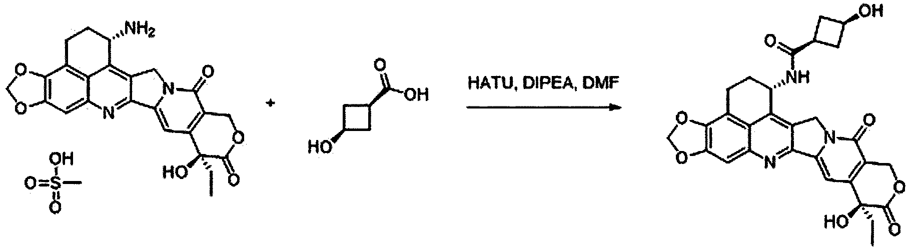
U_AN_ACID: m/z 546.2 [M+H]⁺
¹H NMR (400 MHz, DMSO-d6) δ 8.39 (d, J = 8.8 Hz, 1H), 7.41 (s, 1H), 7.23 (s, 1H), 6.48 (s, 1H), 6.29 (d, J = 3.3 Hz, 2H), 5.57 - 5.49 (m, 1H), 5.46 - 5.35 (m, 2H), 5.17 - 4.99 (m, 3H), 3.99 - 3.88 (m, 1H), 3.04 (q, J = 6.4 Hz, 2H), 2.46 - 2.34 (m, 2H), 2.32 - 2.24 (m, 1H), 2.13 - 2.00 (m, 4H), 1.92 - 1.79 (m, 2H), 0.87 (t, J = 7.3 Hz, 3H).

### Example 1-8. Synthesis of the Camptothecin Derivative of Chemical Formula 9 (Compound 9)

(1R,3R)-3-hydroxycyclobutane-1-carboxylic acid (21 mg, 0.18 mmol) was dissolved in N,N-dimethylformamide (1 mL), and HATU (69 mg, 0.18 mmol) was added. The reaction mixture was stirred for 20 minutes. 0.55 mL of this solution was added to a suspension prepared by dissolving (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione methanesulfonate (49 mg, 90 µmol) and DIPEA (47 µL, 0.27 mmol) in N,N-dimethylformamide (2.5 mL). The mixture was stirred at room temperature for 30 minutes. The reaction mixture was purified by acidic preparative MPLC (Luna10-50), and the product fractions were lyophilized to obtain a yellow solid. (Yield: 29 mg, 59%)

U_AN_ACID: m/z 546.2 [M+H]⁺

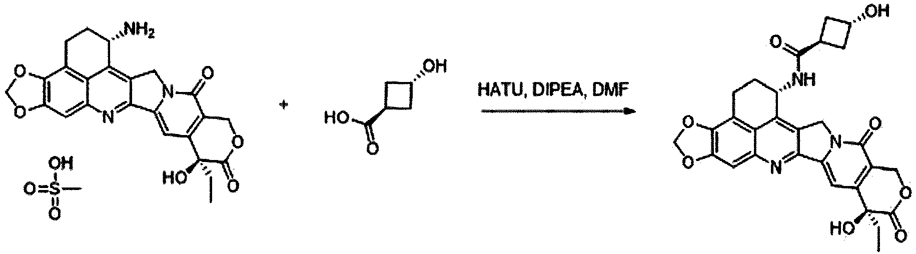

¹H NMR (400 MHz, DMSO-d6) δ 8.36 (d, J = 8.8 Hz, 1H), 7.40 (s, 1H), 7.23 (s, 1H), 6.47 (s, 1H), 6.28 (d, J = 4.3 Hz, 2H), 5.58 - 5.49 (m, 1H), 5.46 - 5.34 (m, 2H), 5.26 - 4.82 (m, 3H), 4.41 - 4.30 (m, 1H), 3.11 - 2.95 (m, 2H), 2.95 - 2.84 (m, 1H), 2.47 - 2.34 (m, 2H), 2.13 - 1.95 (m, 4H), 1.92 - 1.76 (m, J = 7.4 Hz, 2H), 0.87 (t, J = 7.3 Hz, 3H).

### Example 1-9. Synthesis of the Camptothecin Derivative of Chemical Formula 10 (Compound 10)

(R)-3-hydroxybutyric acid (19 mg, 0.18 mmol) was dissolved in N,N-dimethylformamide (1 mL), and HATU (69 mg, 0.18 mmol) was added. The reaction mixture was stirred at room temperature for 20 minutes. 0.55 mL of this solution was added to a suspension prepared by dissolving (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione methanesulfonate (49 mg, 90 µmol) and DIPEA (47 µL, 0.27 mmol) in N,N-dimethylformamide (2.5 mL). The mixture was stirred at room temperature for 30 minutes. The reaction mixture was diluted with 2 mL of DMSO and filtered through a nylon filter. The filter was washed twice with 4 mL of DMSO. All fractions were individually purified by acidic preparative MPLC (Luna10-50), combined, and lyophilized to obtain a white solid. (Yield: 25 mg, 52%) 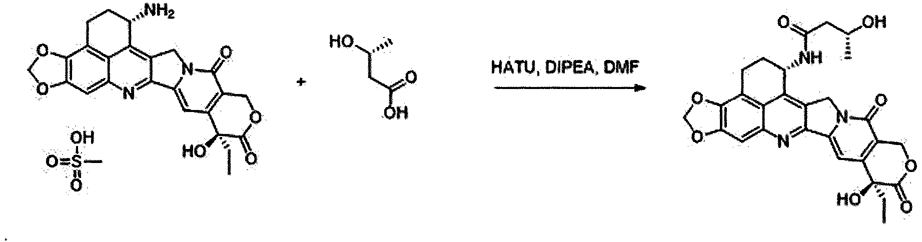
U_AN_ACID: m/z 534.2 [M+H]⁺
¹H NMR (400 MHz, DMSO-d6) δ 8.41 (d, J = 8.8 Hz, 1H), 7.42 (s, 1H), 7.24 (s, 1H), 6.49 (s, 1H), 6.30 (d, J = 6.1 Hz, 2H), 5.59 - 5.47 (m, 1H), 5.41 (s, 2H), 5.26 - 5.05 (m, 2H), 4.65 (d, J = 4.7 Hz, 1H), 4.11 - 3.98 (m, 1H), 3.08 - 2.96 (m, 2H), 2.32 - 2.17 (m, 2H), 2.15 - 1.99 (m, 2H), 1.92 - 1.79 (m, 2H), 1.08 (d, J = 6.2 Hz, 3H), 0.87 (t, J = 7.3 Hz, 3H).

### [Example 2]

### Preparation of Antibody-Drug Conjugate

The compound of Chemical Formula 2 according to the present disclosure was conjugated to trastuzumab through the linker of Chemical Formula 11 to prepare an antibody-drug conjugate comprising the compound of the present disclosure, and the anticancer efficacy thereof was tested.

The preparation of the antibody-drug conjugate was carried out as follows.

Trastuzumab was buffer-exchanged into a reduction buffer (150 mM NaCl, 50 mM histidine, pH 6.0) using a PD-10 desalting column. The antibody (27.5 µM) was treated with 825 µM TCEP at 25°C for 2 hours to reduce the disulfide bonds of the antibody.

Thereafter, excess TCEP was removed using a PD-10 desalting column. In a reaction buffer (25 mM histidine, pH 6.0) containing 15% DMSO, the drug-linker of Chemical Formula 2a comprising the linker of Chemical Formula 11 (165 µM) was reacted with the reduced antibody (13.8 µM) at 25°C for 1 hour to perform the conjugation reaction.

At this time, the drug-to-antibody ratio (DAR) was about 8. After completion of the conjugation reaction, excess drug-linker was removed using a PD-10 desalting column to obtain the final antibody-drug conjugate.

### [Example 3]

### Evaluation of Anticancer Effect of Compound 2

### 3-1. Evaluation of Cell Viability of Compound 2

In this example, in order to evaluate the anticancer efficacy of the compound of Chemical Formula 2 obtained according to Example 1-1, the effects thereof were examined in FaDu and NHEK (Normal Human Epidermal Keratinocytes).

FaDu cells and NHEK cells were seeded at 3,000 cells per well in a 96-well plate and incubated at 37°C in 5% CO₂. After 24 hours, 100 µL of the drug at nine concentrations (starting from 1000 nM and serially diluted at 1/5 intervals) was added to the cells. At this time, the compound of Chemical Formula 2 according to the present disclosure was treated (Dxd was used as a control). After incubation for 3 days at 37°C in 5% CO₂, 100 µL of CellTiter-Glo reagent (CellTiter-Glo^{®} Luminescent Cell Viability Assay kit, Promega, G7571) was added to each well and mixed by pipetting. After incubation for 10 minutes at room temperature (RT), luminescence was measured. When the luminescence value at 0 drug concentration was taken as 100%, the concentration showing 50% luminescence was defined as the IC₅₀ value.

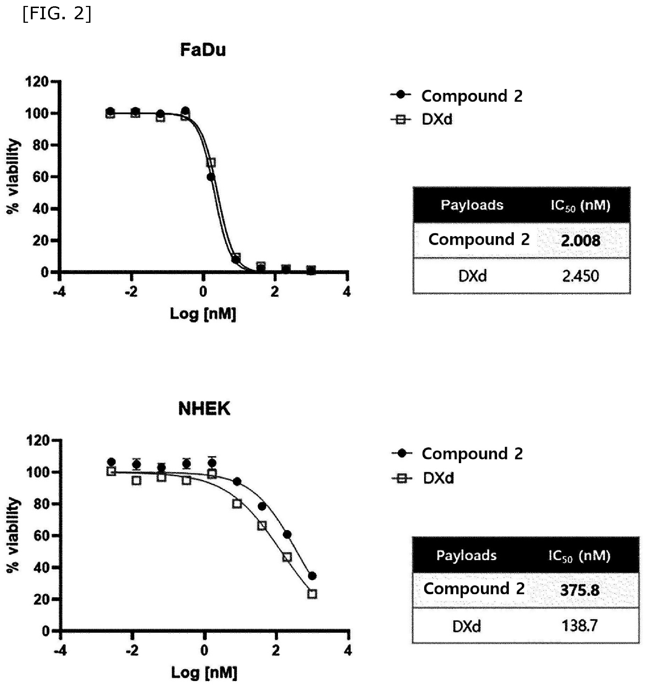

As shown in FIG. 2, Compound 2 (the compound of Chemical Formula 2) of the present disclosure exhibited excellent cytotoxic efficacy in FaDu cells. In addition, Compound 2 showed relatively low cytotoxicity in NHEK (Normal Human Epidermal Keratinocytes) cells, thereby confirming superior safety compared to Dxd.

### 3-2. Evaluation of Cell Viability of Compound 2

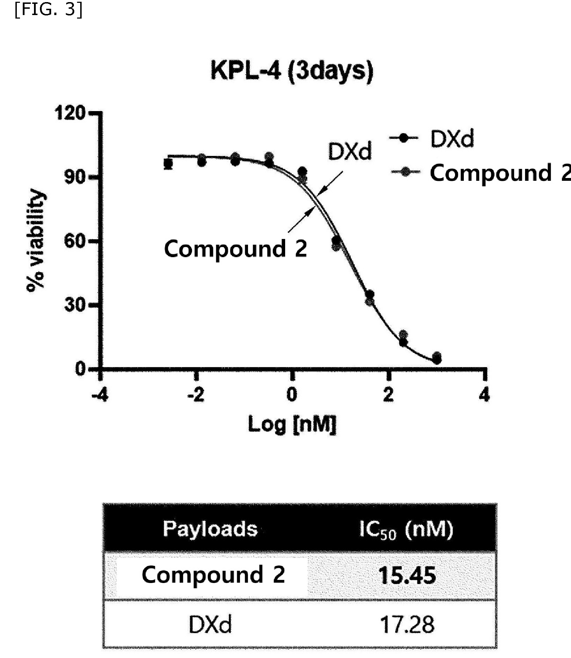

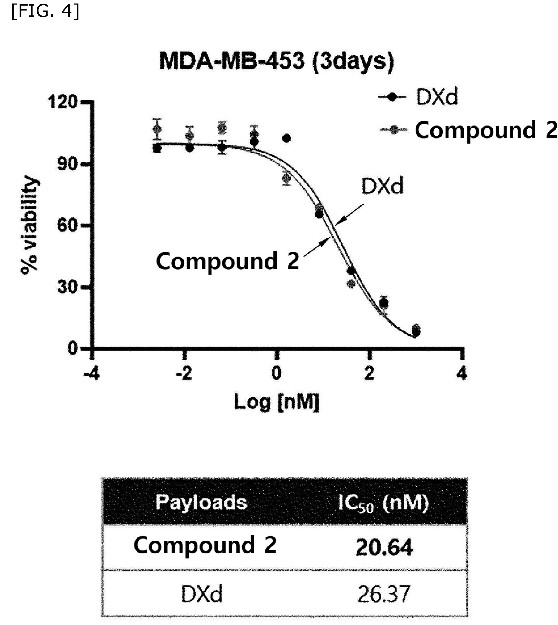

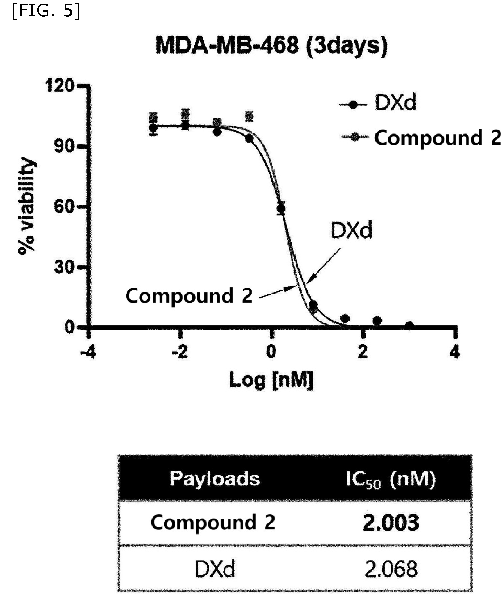

Using the same method as described in Example 3-1, the cancer cell lines KPL-4, MDA-MB-453, and MDA-MB-468 were seeded and incubated, and after 24 hours, Compound 2 (the compound of Chemical Formula 2) was treated. Referring to FIGS. 3, 4, and 5, when Compound 2 was evaluated as a single agent in various cancer cell lines, Compound 2 exhibited cytotoxic efficacy similar to or superior to that of DXd.

### 3-3. Bystander Killing Effect of Compound 2-Based ADC

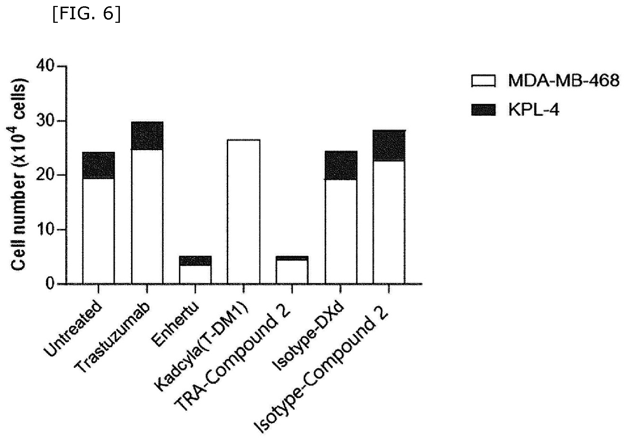

The bystander killing effect of the ADC prepared according to Example 2 (Compound 2-linker-trastuzumab, TRA-Compound 2 in FIG. 6) was evaluated.

Using the same method as described in Example 3-1, the cancer cell lines KPL-4 and MDA-MB-468 were seeded and incubated, and after 24 hours, the compound of Chemical Formula 2 (Compound 2) was treated. As controls, an untreated group, Trastuzumab, Enhertu, Kadcyla (T-DM1), isotype-DXd, and isotype-Compound 2 were each treated. After incubation for 5 days at 37°C in 5% CO₂, the cells were stained with an anti-HER2 FITC antibody, and the total cells were diluted in buffer. The total number of cells was counted using a flow cytometry instrument (Beckman Coulter), and the number of cells exhibiting FITC fluorescence was measured.

Referring to FIG. 6, the Compound 2-based ADC exhibited a bystander killing effect comparable to that of Enhertu, and the efficacy thereof was superior to that of Kadcyla (T-DM1). In addition, in the HER2-positive KPL-4 cell line, it showed superior cytotoxic efficacy compared to Enhertu.

### 3-4. Evaluation of Cell Viability of Compound 2-Based ADC

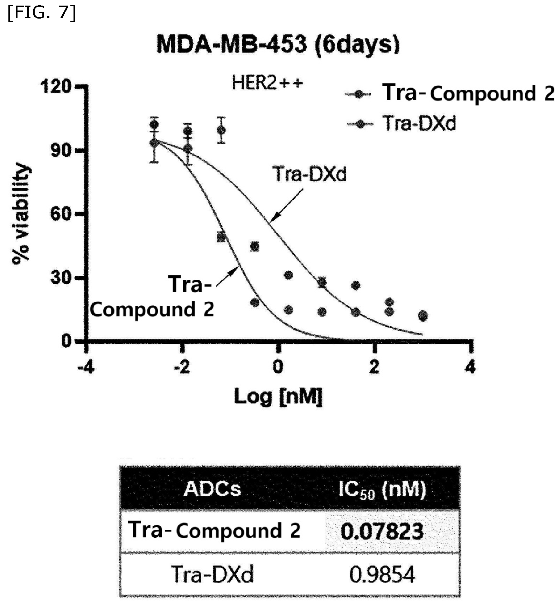

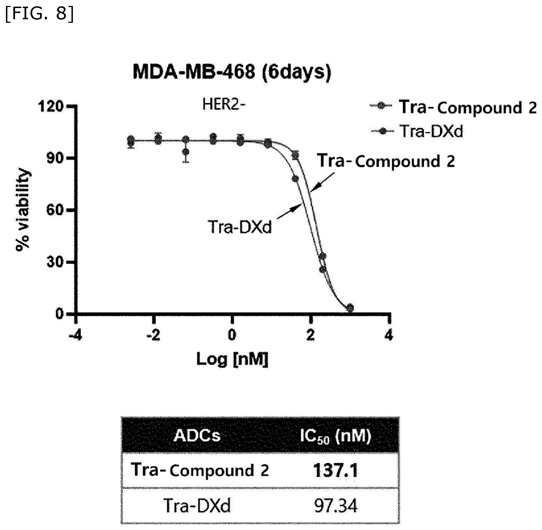

Using the same method as described in Example 3-1, the cancer cell lines MDA-MB-453 and MDA-MB-468 were seeded and incubated, and after 24 hours, the Compound 2-based ADC was treated. When the HER2-targeting ADC based on Compound 2, i.e., Tra-Compound 2, was evaluated in various cancer cell lines having different levels of HER2 expression, it exhibited superior cytotoxic efficacy compared to Tra-DXd in HER2-positive cell lines. In addition, the Compound 2-based ADC showed low cytotoxicity in HER2-negative cell lines, thereby confirming improved safety compared to DXd (see FIGS. 7 and 8). That is, the Compound 2-based ADC prepared according to the present disclosure demonstrated superior selectivity toward cancer cells and higher cytotoxicity compared to the conventional drug (DXd), even when the same antibody was used.

Overall, according to the above results, the compound belonging to Chemical Formula 1 of the present disclosure not only exhibits cytotoxicity against cancer cells as a single agent, but also exhibits high cytotoxicity against cancer cells in the form of an ADC conjugated with a linker-antibody. In particular, when prepared in the form of an ADC, the compound of Chemical Formula 2 showed significant cytotoxicity compared to the conventional drug and high selectivity toward target cancer cells, thereby confirming that it may be used as an excellent anticancer agent.

The present disclosure has been described above with reference to preferred embodiments thereof. Those skilled in the art to which the present disclosure pertains will understand that the present disclosure may be implemented in modified forms without departing from the essential characteristics of the present disclosure. Therefore, the disclosed embodiments should be considered in a descriptive sense only and not for purposes of limitation. The scope of the present disclosure is defined by the appended claims rather than by the foregoing description, and all differences falling within the equivalent scope thereof shall be construed as being included in the present disclosure.

## Claims

1. A compound represented by Chemical Formula 1 below, an isomer thereof, a pharmaceutically acceptable salt thereof, or a solvate thereof:
wherein, R is selected from the group consisting of hydrogen; halogen; substituted or 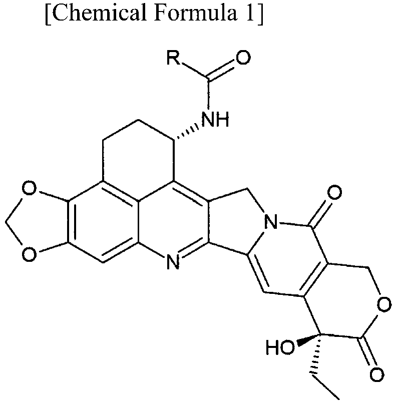 unsubstituted C1-C10 alkyl; substituted or unsubstituted C1-C10 alkoxy; substituted or unsubstituted C2-C10 alkenyl; C2-C10 alkynyl; substituted or unsubstituted C3-C10 cycloalkyl; substituted or unsubstituted C2-C10 heterocycloalkyl; substituted or unsubstituted C6-C10 aryl; substituted or unsubstituted C2-C10 heteroaryl; C1-C10 monoalkylamino or dialkylamino; nitro; and cyano.

2. The compound of claim 1,
wherein the compound is a compound represented by Chemical Formula 2 below, an isomer thereof, a pharmaceutically acceptable salt thereof, or a solvate thereof:

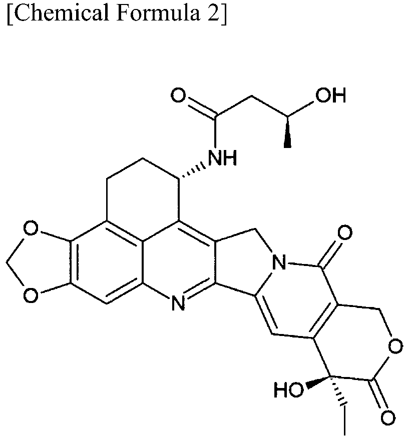

3. A carrier-drug conjugate comprising:
a compound of Chemical Formula 1, a pharmaceutically acceptable salt thereof, or a solvate thereof:
wherein, R is selected from the group consisting of hydrogen; halogen; substituted or 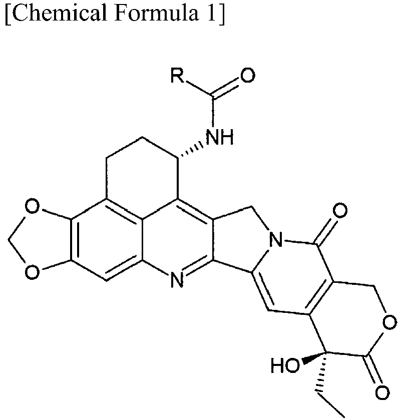 unsubstituted C1-C10 alkyl; substituted or unsubstituted C1-C10 alkoxy; substituted or unsubstituted C2-C10 alkenyl; C2-C10 alkynyl; substituted or unsubstituted C3-C10 cycloalkyl; substituted or unsubstituted C2-C10 heterocycloalkyl; substituted or unsubstituted C6-C10 aryl; substituted or unsubstituted C2-C10 heteroaryl; C1-C10 monoalkylamino or dialkylamino; nitro; and cyano.

4. The carrier-drug conjugate of claim 3,
wherein the compound of Chemical Formula 1 is a compound represented by Chemical Formula 2 below:

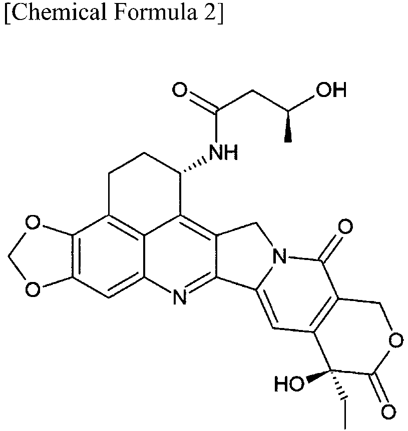

5. The carrier-drug conjugate of claim 3, wherein the carrier is an antibody, a peptide, a lipibody, or an aptamer.

6. The carrier-drug conjugate of claim 3, wherein the carrier is conjugated to the drug via a linker.

7. The carrier-drug conjugate of claim 6, wherein the linker comprises GGFG amino acid sequence.

8. The carrier-drug conjugate of claim 5,
wherein the carrier is an antibody, peptide, lipibody, or aptamer that specifically binds to one or more antigens selected from the group consisting of:
4-1BB, 5T4, integrin, activin, amyloid beta, angiopoietin (angiopoietin-1 or angiopoietin-2), angiopoietin-like 3, B cell maturation antigen (BCMA), B-cell activating factor (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, Claudin-18, c-Met, CSF-1, CTLA4, DLL3, EGF receptor, hemophilia factor, Fc receptor, FGF23, folate receptor, GD2, glucocorticoid-induced TNF receptor (GITR), Glypican 3, GM-CSF, HER2, HER3, TROP2, hepatocyte growth factor (HGF), interferon receptor, interferon gamma, IgE, IGF-1 receptor, interleukin-1, interleukin-2 receptor, interleukin-4, interleukin-4 receptor, interleukin-5, interleukin-5 receptor, interleukin-6, interleukin-6 receptor, interleukin-8, interleukin-12/23, interleukin-13, interleukin-17A, interleukin-17 receptor A, interleukin-23, interleukin-31 receptor, interleukin-36 receptor, lymphocyte-activation gene 3 (LAG3), lysyl oxidase homolog 2 (LOXL2), mesothelin, Mucin-1, Mucin-16, Nectin-4, nerve growth factor (NGF), OX40, proprotein convertase subtilisin/kexin type 9 (PCSK9), PD-1, PD-L1, phospholipase C, Receptor activator of nuclear factors kappa B ligand(RANKL), Tyrosine-protein kinase transmembrane receptor(ROR1), Sialic acid binding ig-like lectin 15 (Siglec-15), transforming growth factor beta (TGFβ), T-cell immunoreceptor with immunoglobulin and ITIM domain (TIGIT), T cell immunoglobulin and mucin-domain containing-3 (Tim-3), tissue factor, tissue factor pathway inhibitor (TFPI), TROP-2, tumor necrosis factor (TNF), thymic stromal lymphopoietin (TSLP), Colony stimulating factor 1 receptor (CSF1R), vascular endothelial growth factor (VEGF), VEGF receptor, and von Willebrand factor (vWF).

9. The carrier-drug conjugate of claim 5, wherein the carrier is an antibody.

10. The carrier-drug conjugate of claim 9,
wherein the antibody is selected from the group consisting of:
Urelumab, Utomilumab, Bebtelovimab, Aducanumab, Bapineuzumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, Navicixizumab.

11. A drug-linker comprising:
a compound of Chemical Formula 1, a derivative thereof, an isomer thereof, a pharmaceutically acceptable salt thereof, or a solvate thereof; and a carrier linked to the compound:
wherein, R is selected from the group consisting of hydrogen; halogen; substituted or 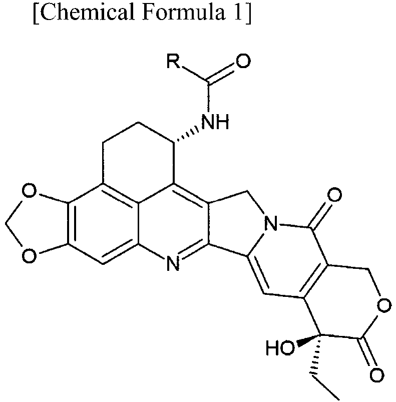 unsubstituted C1-C10 alkyl; substituted or unsubstituted C1-C10 alkoxy; substituted or unsubstituted C2-C10 alkenyl; C2-C10 alkynyl; substituted or unsubstituted C3-C10 cycloalkyl; substituted or unsubstituted C2-C10 heterocycloalkyl; substituted or unsubstituted C6-C10 aryl; substituted or unsubstituted C2-C10 heteroaryl; C1-C10 monoalkylamino or dialkylamino; nitro; and cyano.

12. The drug-linker of claim 11, wherein the linker comprises GGFG amino acid sequence.

13. A pharmaceutical composition for preventing or treating cancer, comprising:
the compound of claim 1, the carrier-drug conjugate of claim 3, or the drug-linker of claim 11.

14. The pharmaceutical composition of claim 13,
wherein the cancer is one or more selected from the group consisting of:
pseudomyxoma peritonei, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, bile duct cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, cardiac cancer, duodenal cancer, malignant soft tissue cancer, malignant bone cancer, malignant lymphoma, malignant mesothelioma, malignant melanoma, ocular cancer, vulvar cancer, ureter cancer, urethral cancer, cancer of unknown primary, gastric lymphoma, gastric cancer, gastric neuroendocrine tumor, gastrointestinal stromal tumor, Wilms tumor, breast cancer, triple-negative breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal neuroendocrine tumor, vaginal cancer, spinal cord cancer, acoustic neuroma, pancreatic cancer, salivary gland cancer, Kaposi sarcoma, Paget disease, tonsillar cancer, squamous cell carcinoma, lung adenocarcinoma, lung cancer, lung squamous cell carcinoma, skin cancer, anal cancer, rhabdomyosarcoma, laryngeal cancer, pleural cancer, hematologic cancer, and thymic cancer.
